# EUROPEAN PATENT APPLICATION

(11) **EP 1 745 780 A1**
(43) Veröffentlichungstag der Anmeldung: **24.01.2007**
(21) Anmeldenummer: 05735316.1
(22) Anmeldetag: 31.03.2005
(51) Int. Cl.: A61K 31/192, A61P 35/00

(54) **MEDIZINISCHES PRÄPARAT**

(30) Priorität: 22.04.2004 RU 2004112187
(71) Anmelder: Kutushov, Mikhail Vladimirovich, Moscow, 125414 (RU); Germanov, Evgeny Pavlovich, Moscow, 121085 (RU)
(72) Erfinder: KUTUSHOV, Mikhail Vladimirovich, Moscow, 125414 (RU)
(74) Vertreter: Jeck, Anton
(86) Internationale Anmeldenummer: PCT/RU2005/000155
(87) Internationale Veröffentlichungsnummer: WO 2005/102302

(57) **Zusammenfassung**

Die Erfindung betrifft die Medizin, insbesondere medizinische Breitspektrum-Präparate. Die Erfindung basiert auf der Verwendung einer 4-chlor-2-methylphenoxysauren Säure der Formel (I) und von deren pharmakologisch annehmbaren Derivaten, beispielsweise von entsprechenden Alkali-Metall-Salzen dieser Säure und dieser Derivate in Form von pharmazeutischen Präparaten, die immunomodulierende, Antientzündungs- und Antitumoreigenschaften sowie antivirale Aktivitäten aufweisen. Das Breitspektrum der therapeutischen Arbeitsweisen des medizinischen Präparats gemäß der Erfindung und die Tatsache, dass dieses Präparat keine Nebenwirkungen erzeugt, sind durch Studien nachgewiesen.

## Beschreibung

Die Erfindung gehört zum medizinischen Bereich, insbesondere zu Heilmitteln mit breitem Wirkungsspektrum.

Bekannt ist das Präparat Fluoruracil, das ein weißes oder leicht gelbliches, schwer wasser- und alkohollösliches, kristallines Pulver und ein antimetabolisches Mittel ist. Die antiblastomatöse Aktivität dieses Präparats wird durch dessen Umwandlung in Krebszellen in einen Konkurrenzinhibitor bestimmt, der an der Synthese eines Ferments von Nukleinsäuren teilnimmt (s. M.D. Maschkowskij, Arzneimittel, Moskau, Nowaja Wolna/Neue Welle GmbH, Verleger S.B. Diwow, 2002, Band 2, S. 425).

Das Präparat wird als intravenöse Injektionen bei inoperablen Tumoren und Rezidivtumoren des Magens, Dick- und Mastdarm-, Mamma- und Eierstockkrebs sowie Pankreaskrebs eingesetzt. Das Präparat besitzt aber eine hohe Toxizität und weist eine eventuelle Unterdrückung der Blutbildung, Diarrhoe, Appetitminderung, Erbrechen und geschwürartige Stomatitis bei seiner Anwendung auf. Darüber hinaus ist mit dem Präparat eine Gegenanzeige bei allgemein schwerem Krankenzustand, beim Magen- und Zwölffingerdarmgeschwür und ausgeprägtem Leberversagen verbunden.

Der am nächsten kommende Prototyp ist das Reaferon, das ein rekombinantes α₂-Interferon darstellt, das vom Pseudomonaden-Bakterienstamm produziert wird. Im Genapparat des α₂-Interferon ist ein Gen des leukozytischen, humanen α₂-Interferons eingebaut ist. Das α₂-Interferon wird als poröses Pulver hergestellt, dessen Wasserlösung als intramuskuläre und subkutane Injektionen eingesetzt wird (s. M.D. Maschkowskij, Arzneimittel, Moskau, Nowaja Wolna/Neue Welle GmbH, Verleger S.B. Diwow, 2002, Band 2, S. 323-324).

Das Präparat besitzt eine immunmodulierende, antiblastomatöse und Antivirusaktivität, wirkt effektiv bei der Behandlung der viralen Hepatitis und wird bei der Behandlung von Haarzellenleukämie, Kaposhi-Sarkomen (bei AIDS), Nierenkrebsen, metastasischen Melanomen u.a. eingesetzt. Bei seinem Einsatz sind aber Nebenwirkungen möglich, wie Schauer, allgemeines Unwohlsein, allergische Hautreaktionen, Leuko- und Trombozytopenie.

Die Aufgabe der Erfindung besteht darin, ein Arzneimittel zu schaffen, das ein breites Heilwirkungsspektrum besitzt und keine Nebenwirkungen bei seiner Anwendung in pharmazeutisch vertretbaren Dosen verursacht.

Bekannt ist 4-Chlor-2-Methylphenoxyessigsäure, die eine farblose, wasser- und alkohollöslische, kristalline Substanz darstellt und in der Agronomie als Herbizid unter den Bezeichnungen 2M-4X, MCPA, Metoxon u.a. eingesetzt wird (s. Katalog Schadstoffe in der Industrie, Verlag Chemie, Leningrad, 1976, S. 131).

Durch Versuche wurde die genannte Säure in den inneren Organen und im Blut der Menschen die mit der Herstellung und Anwendung dieser Säure befasst waren, nachgewiesen, wobei sie aus dem Organismus vorwiegend mit dem Urin ausgeschieden wird (s. Bache C.A. et al., Daire Sci., 1964, Vol. 47, S. 93-95).

Nach Empfehlungen des Forschungsinstituts WNIIGINTOKS ist für die Herstellung dieser Säure und ihrer Derivate eine maximal zulässige Konzentration dieser Stoffe in den Produktionsräumen festgelegt. Beispielsweise ist für Natriumsalz 1mg/m³ festgelegt (s. Leitfaden zur Durchführung der Hygieneüberwachung der Arbeitsbedingungen bei der Produktion von 2M-4X-Herbizid [Metoxon], Ufa, 1971).

Ferner wurde aus den biochemischen Untersuchungen der Herbizidwirkung auf Pflanzenwachstumsfaktoren festgestellt, dass die genannte Säure die Energietransformation in der Zelle beeinflusst, die Intensivität der Oxidationsprozesse erhöht und das Phosphorylieren in den Mitochondrien unterdrückt. Dabei wird durch eine Herbizidkumulation in den Membranen eine Störung des mit den Membranen des Proteinfaktors zusammenhängenden Komplexes verursacht. Die Aktivität der RNA-Polymerase wird bestimmt, die mittels einer Transkription bestimmte mRNAs synthesiert. Mit einer Änderung der funktionellen Aktivität der Membranen und der damit zusammenhängenden Enzyme können Herbizide mit Gameteigenschaften eine Induktion der Synthese von mRNAs auslösen, die für die Produktion der Enzyme und folglich der Fermente verantwortlich sind, die die Kohlenhydratkomponenten der Membranen und Zellhüllen umwandeln (s. Hardin J.W., Morre D.J., Lembi C.A., Enhancement of soybean R'NA polymerase activity by a factor released by auxin from plasma-membrane; Proc. Naitl. Acad. Sci., USA, 1972, Vol. 69, N 11, S. 31146-31150).

Diese Daten führen zu der Annahme, dass einige Herbizide auf den menschlichen Organismus ähnlich einwirken.

Die gestellte Aufgabe wurde durch ein Arzneimittel gelöst, das immunmodulierende, entzündungshemmende und antiblastomatöse Eigenschaften sowie eine Antivirusaktivität besitzt und dadurch gekennzeichnet ist, dass es die 4-Chlor-2-Methylphenoxyessigsäure mit Strukturformel: 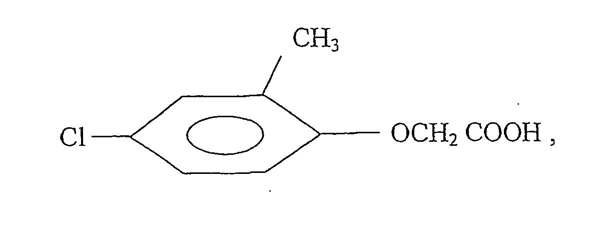 und/oder ihre pharmakologisch vertretbaren Derivate, beispielsweise Salze der Alkalimetalle oder Gemische dieser Salze bzw. Gemische aus 4-Chlor-2-Methylphenoxyessigsäure und diesen Derivaten enthält.

Das Derivat der 4-Chlor-2-Methylphenoxyessigsäure stellt dabei entweder ein Kalium- oder Natrium- oder Lithiumsalz dieser Säure dar.

Darüber hinaus stellt das MCPA-Derivat entweder ein Gemisch aus Kalium- und Natriumsalz der Säure im Verhältnis von 1,0-98,0 Gew.-% Kaliumsalz und restlichem Natriumsalz oder ein Gemisch aus 1,0-98,0 Gew.-% Kaliumsalz und restlichem Lithiumsalz oder ein Gemisch aus 1,0-98,0 Gew.-% Natriumsalz und restlichem Lithiumsalz oder ein Gemisch aus 1,0-0,55 Gew.-% Kaliumsalz, 1,0-0,85 Gew.-% Natriumsalz und restlichem Lithiumsalz dar.

Die 4-Chlor-2-Methylphenoxyessigsäure wird dabei mit ihren Derivaten in einem Verhältnis von 1,0-98,0 Gew.-% MCPA und restlichen Derivaten gemischt.

Das Heilmittel mit immunmodulierenden, entzündungshemmenden und antiblastomatösen Eigenschaften sowie Antivirusaktivität enthält 4-Chlor-2-Methylphenoxyessigsäure und/oder ihre pharmakologisch vertretbaren Derivate, beispielsweise Salze von Alkalimetallen dieser Säure oder Gemische dieser Salze bzw. Gemische aus MCPA und deren Derivate.

Das Derivat der 4-Chlor-2-Methylphenoxyessigsäure stellt dabei entweder ein Kalium- oder Natrium- oder Lithiumsalz dieser Säure dar.

Darüber hinaus stellt das MCPA-Derivat entweder ein Gemisch aus Kalium- und Natriumsalz der Säure in einem Verhältnis von 1,0-98,0 Gew.-% Kaliumsalz und restlichem Natriumsalz oder ein Gemisch aus 1,0-98,0 Gew.-% Kaliumsalz und restlichem Lithiumsalz oder ein Gemisch aus 1,0-98,0 Gew.-% Natriumsalz und restlichem Lithiumsalz oder ein Gemisch aus 1,0-0,55 Gew.-% Kaliumsalz, 1,0-0,85 Gew.-% Natriumsalz und restlichem Lithiumsalz dar.

Die 4-Chlor-2-Methylphenoxyessigsäure wird dabei mit ihren Derivaten in einem Verhältnis von 1,0-98,0 Gew.-% MCPA und restlichen Derivaten gemischt.

Die 4-Chlor-2-Methylphenoxyessigsäure wird mittels einer Chlorierung von o-Kresol, einer weiteren Reaktion mit Chloressigsäure und einer Ausscheidung des Zielproduktes aus dem erhaltenen Gemisch, beispielsweise durch Umkristallisieren, gewonnen.

Salze von Alkalimetallen werden in folgender Form erhalten: 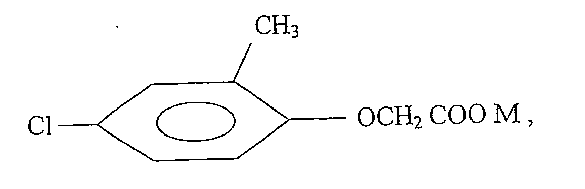 wobei M = K, Na, Li ist,
beispielsweise mittels der Wechselwirkung einer Wasserlösung der Säure entweder mit Kalium- oder Natrium- oder Lithiumhydroxid und benötigter Gemische durch Auswahl bestimmter Inhaltsstoffe in einem vorgegebenen Verhältnis.

Durchgeführte Untersuchungen zeigten, dass die 4-Chlor-2-Methylphenoxyessigsäure und ihre pharmakologisch vertretbaren Derivate in Form von Alkalimetallsalzen sowie entsprechende Gemische dieser Salze enthaltene Heilmittel die Zell- und Humoralschutzfaktoren fördern, eine entzündungshemmende und antiblastomatöse Wirkung haben, die sich beispielsweise in einem vom Erfinder festgestellten und als "disymmetrisch" bezeichneten therapeutischen Effekt äußert. Dieser Effekt prägt sich in der an unterschiedlichen Körperpunkten gemessenen Temperatur aus, beispielsweise 2-4° C in 0,5-2 Stunden nach der Heilmitteleinführung (s. M.W. Kutuschow, Krebs, Heilung ist möglich, Verlag Newa, St-Petersburg, 2003). Das Heilmittel besitzt eine Antivirusaktivität.

Dies wird durch Labor- sowie Patientenuntersuchungen der Blutwerte des Leukozyten- und Lymphsystems der Patienten vor und nach der Einführung des Präparates bestätigt.

Das auf der Basis des Arzneimittels gewonnene Präparat kann als Pulver peroral oder als Tabletten während oder nach dem Essen eingenommen werden, wobei seine Einnahme in einem breiten Dosisbereich (von 10 mg bis 4 g) keine allergischen Reaktionen und andere Nebenwirkungen verursacht und seine Effektivität (bei der Behandlung entsprechender Krankheiten) nicht geringer als die des Prototyps ist.

Untersuchungsergebnisse des Wirkungsmechanismus des Arzneimittels gemäß der vorliegenden Erfindung werden in den folgenden Beispielen 1-6 aufgeführt, und die Einsatzmöglichkeiten des Arzneimittels werden durch die folgenden Beispiele 7-12 bestätigt.

### Beispiel 1

In Prüfgläser einer Versuchsgruppe mit Krebszellen PC-3 (Prostatakarzinom) in einer Pufferlösung (10 ml) wird das Arzneimittel in einer Verdünnung von 10⁻⁵ mmol/l in Form von 4-Chlor-2-Methylphenoxyessigsäure, ihren Kalium-, Natrium- und Lithiumsalzen, Gemischen von Kalium- und Natriumsalz, Kalium- und Lithiumsalz, Natrium- und Lithiumsalz, Kalium-, Natrium- und Lithiumsalz (im gleichen Verhältnis der Inhaltsstoffe) und Gemischen aus dieser Säure und Kalium-, Natrium- und Lithiumsalz (im Verhältnis 25 Gew.-% Säure und restlichem Salz) eingeführt.

Ferner werden die Präparate Adriamycin, Reaferon und physiologische Kochsalzlösung in Prüfgläser mit demselben Krebszellenpool eingeführt.

Dabei wurde eine Kontrollgruppe mit derselben Prüfgläseranzahl und denselben Komponenten außer Krebszellen gebildet, und in die Prüfgläser dieser Gruppe wurden Fibroblasten statt Krebszellen eingeführt.

Nach einer Thermostatisierung der o.g. Prüfgläser im Laufe von 24 Stunden bei einer Temperatur von 37° C wurden die Inhalte analysiert.

### Die gewonnenen Ergebnisse zeigten:

In der Versuchsgruppe mit dem Heilmittel wurden die Mitochondrienmembranen praktisch vollständig zerstört, wobei das Erscheinungsbild für alle Zusammensetzungen ungefähr gleich war. In den Prüfgläsern mit Adriamycin wurde eine Membranschwellung, mit Reaferon eine Membranschwellung mit teilweiser (ca. 60 %) Zerstörung beobachtet; die Zellen in den Prüfgläsern mit physiologischer Kochsalzlösung zeigten keine Änderung.

In den Prüfgläsern der Kontrollgruppe sind die Mitochondrienmembranen geschwollen, ihre Vollständigkeit ist aber erhalten.

Die gewonnenen Daten weisen nach, dass das Heilmittel gemäß der vorliegenden Erfindung eine Zerstörung der Mitochondrienmembranen von Krebszellen verursacht, aber die Struktur der Normalzellen nicht zerstört. Anders gesagt, bildet das Arzneimittel verstärkt die Fähigkeit aus, Fermente zu bilden, die eine Krebszellenapoptose verursachen.

### Beispiel 2

In vier Prüfgläser einer Versuchsgruppe mit Krebszellen MCF-7 (Brustdrüsenadenokarzinom) in einer Pufferlösung (10 ml) wurde das Arzneimittel in einer Verdünnung von 10⁻⁵ mmol/l in Form von 4-Chlor-2-Methylphenoxyessigsäure und Gemischen dieser Säure mit Kalium-, Natrium- und Lithiumsalzen in zwei Verhältnissen: 1.) 25 Gew.-% Säure, 25 Gew.-% Kalium-, 25 Gew.-% Natrium- und 25 Gew.-% Lithiumsalz; 2.) 70 Gew.-% Säure, 10 Gew.-% Kalium-, 10 Gew.-% Natrium- und 10 Gew.-% Lithiumsalz; 3.) 10 Gew.-% Säure, 10 Gew.-% Kalium-, 55 Gew.-% Natrium- und 25 Gew.-% Lithiumsalz eingeführt.

Für die Kontrollgruppe mit demselben Krebszellenpool werden in ein Prüfglas eine physiologische Kochsalzlösung und in die restlichen Prüfgläser Adriamycin und Reaferon eingeführt.

In den beiden Prüfgläsergruppen werden Titer von Zytochrom C bestimmt, die 1:14000 ausmachen.

Nach einer Thermostatisierung der o.g. Prüfgläser im Laufe von 24 Stunden bei einer Temperatur von 37° C wurden die Inhalte analysiert.

### Die gewonnenen Ergebnisse zeigten:

In den Prüfgläsern der Versuchsgruppe mit dem eingeführten Arzneimittel beträgt der Titer von Zytochrom C im Prüfglas mit MCPA 1:2000, und in den Prüfgläsern mit dem Arzneimittel in Form von Gemischen dieser Säure mit entsprechenden Salzen beträgt der Titer 1: (1950-2250).

In den Prüfgläsern mit Adriamycin und Reaferon beträgt der Titer 1:12000 bzw. 1:9600. Im Prüfglas mit der physiologischen Kochsalzlösung wird keine besondere Titeränderung beobachtet.

Die gewonnenen Ergebnisse führen zu der Annahme, dass das Arzneimittel eine Freisetzung eines "aggressiven" Proteins von Zytochrom C aus den Membranen der Krebszellenmitochondrien fördert, das Apoptose dieser Zellen verursacht, wobei es den Zerstörungsmechanismus der Desoxyribonukleinsäure durch Kaspasen auslöst (s. Wilson, B.E., Mochon, E.A., Boxer, L.M, Induction of blc-2 expression by phosphorylated CREB proteins during B-cell activation and rescue from apoptosis, Mol. Cell. Biol., 1996, Vol.16, S. 5546-5556).

### Beispiel 3

In ein Prüfglas (10 ml) mit Transthyretin (Protein) des Blutplasmas (pH = 7,0) in einer Konzentration von 0,05 mmol/ml wurden vier Tropfen 0,1 %-ige MCPA-Wasserlösung eingeführt. Das Prüfglas wurde bei einer Temperatur von 37° C thermostatiert. Nach 15 Minuten wurden die Konzentration von Transthyretin und der pH-Wert des Blutplasmas gemessen. Transthyretin wurde nicht gefunden, und der pH-Wert war 6,0.

Daraus folgt, dass das Arzneimittel auf die Proteindenaturierung nicht als Säure, sondern als Präparat wirkt, das die Polymerstruktur ändert. Diese Eigenschaft verursacht den therapeutischen Effekt bei der Behandlung von bösartigen Geschwülsten.

### Beispiel 4

In ein Prüfglas mit β-Amyloid (Protein) des Blutplasmas (pH = 7,4) mit einer Konzentration von 0,1 mmol/ml werden drei Tropfen 0,1 %-ige Natriumsalzwasserlösung (als Ö.F.1 bezeichnet) eingeführt. Das Prüfglas wurde bei einer Temperatur von 37° C thermostatiert. Nach 15 Minuten wurden die Proteinkonzentration und der pH-Wert des Blutplasmas gemessen. Die Proteinkonzentration betrug 0,03 mmol/ml, und der pH-Wert betrug 7,2.

Daraus folgt, dass das Präparat O.F.1 die Denaturierung des Amyloid-Vorläuferproteins und seine Umwandlung in β-Amyloid verhindert. Diese Eigenschaft bewirkt den therapeutischen Effekt bei der Behandlung von Amyloidosen und Krebs.

### Beispiel 5

Das Blut eines Sarkomkranken (10 ml) wurde zentrifugiert. Ein Plasmaanteil wurde getrocknet und im Polarisationsmikroskop aufgenommen. In den restlichen Plasmaanteil wurde eine Wasserlösung von Lithiumsalz der MCPA-Säure eingeführt, und das Gemisch wurde ebenfalls im Polarisationsmikroskop aufgenommen.

Ergebnisse: In der ersten Zusammensetzung erfolgte eine Lichtdiffusion, und in der zweiten Zusammensetzung erfolgte eine doppelte Strahlenbrechung. Diese Daten sprechen dafür, dass das Arzneimittel die wegen des Krebses geänderte Proteinfaltung in Ordnung bringt.

### Beispiel 6

Ein Filtrat aus dem Melanom 16 (10 ml) wurde in einer Quarzküvette untergebracht und im Polarisationsmikroskop aufgenommen. Danach wurde Natriumsalz (O.F.1) in das Filtrat eingebracht und eine wiederholte Aufnahme im Polarisationsmikroskop durchgeführt.

Ergebnisse: Auf dem Filtratbild ohne Präparat ist die Lichtpolarisation gering, und auf dem Bild mit Präparat wurde eine ausgeprägte Polarisation des durch die Zusammensetzung hindurchgehenden Strahls beobachtet. Die gewonnenen Daten weisen nach, dass das Arzneimittel strukturelle Änderungen im Eiweiß von Krebszellen verursacht.

### Beispiel 7

Bei Mäusen der Rasse B-57 wurde die Reaktion auf Arzneimittel in Form von 4-Chlor-2-Methylphenoxyessigsäure und deren Kalium-, Natrium- und Lithiunsalzen geprüft.

Die Versuchsgruppe umfasste 40 Mäuse und wurde in 4 Untergruppen je 10 Mäusen eingeteilt. Die Kontrollgruppe umfasste 10 Mäuse. Die Untergruppen der Versuchsgruppe wurden dabei von der Kontrollgruppe getrennt gehalten. Die Mäuse jeder Untergruppe wurden von 1 bis 10 mit einem Farbmittel, in diesem Fall mit Viride nitens, gekennzeichnet.

Die Versuchsgruppe erhielt das Arzneimittel im Laufe von 10 Tagen: die 1. Untergruppe die 4-Chlor-2-Methylphenoxyesseigsäure, die 2. Untergruppe das Kaliumsalz der Säure, die 3. Untergruppe das Natriumsalz der Säure (Präparat O.F.1) und die 4. Untergruppe das Lithiumsalz der Säure. Dabei erhielten die Mäuse Nr. 1-8 das Präparat als Getränk (in einer Wasserlösung) und Nr. 9-10 als Injektionen in das Bauchfell. In jeder Untergruppe war dabei die Einmaldosis für die Mäuse Nr. 1-2 2 mg, für Nr. 3-4 10 mg, für Nr. 5-6 15 mg, für Nr. 7-8 20 mg und Nr. 9-10 5 mg pro 1 ml Injektionswasser. Die Mäuse der Kontrollgruppe erhielten kein Präparat.

Ergebnisse: Nach zwei Wochen seit der Einführung des Arzneimittels waren alle Mäuse der Versuchs- und Kontrollgruppe am Leben geblieben. Es wurden keine Unterschiede im Verhalten der Versuchs- und Kontrollgruppe beobachtet. Die Mäuse mit geraden Nummern in allen Untergruppen wurden am 21. Tag getötet.

Spuren des Arzneimittels wurden in den Nieren der Nr. 8 in allen Untergruppen der Versuchsgruppe festgestellt. Bei einer histologischen Prüfung wurden keine Unterschiede in der Einwirkung der 4-Chlor-2-Methylphenoxyessigsäure und deren Salze auf den Organismus festgestellt.

Daraus kann mit Rücksicht auf Massenverhältnisse vermutet werden, dass die Einmaldosis von 4,0 g für den menschlichen Organismus vertretbar ist. Genauere Daten können aber erst nach entsprechenden Untersuchungen angegeben werden. Beispiel 8

Die Mäuse der Rasse B-57 der Versuchs- und Kontrollgruppe (je 60 Mäuse) wurden mit Melanom-16 geimpft. Die Versuchs- und Kontrollgruppe wurde in 3 Untergruppen mit je 20 Mäusen eingeteilt. Die 1.-3. Untergruppe der Versuchsgruppe erhielt das Arzneimittel als Getränk in einer Einmaldosis von 5 mg in Wasserlösungen der 4-Chlor-2-Methylphenoxyessigsäure, deren Natriumsalz (Präparat O.F.1) und des Gemisches aus MCPA sowie Kalium- und Natriumsalz in einem Verhältnis von 25:25:50 Gew.-%. Die erste und zweite Untergruppe der Kontrollgruppe erhielt als Getränk Wasserlösungen von Adriamycin und Reaferon in einer Einmaldosis von 5 mg. Die dritte Untergruppe erhielt keine Präparate.

Ergebnisse: Am 36. Tag des Versuchs waren 22 von 30 Mäusen der Versuchsgruppe am Leben geblieben. 4 Mäuse (2 aus der ersten, je eine Maus aus der zweiten und dritten Untergruppe) starben am 23. Tag, 3 Mäuse (eine aus der zweiten und 2 aus der dritten Untergruppe) am 26. Tag und 1 Maus (aus der zweiten Untergruppe) am 30. Tag des Versuchs. 15 überlebende Mäuse (3 aus der ersten, 8 aus der zweiten und 4 aus der dritten Untergruppe) hatten Blastome mit einer Größe von etwa 0,3x0,2 mm. Bei 7 Mäusen (3 aus der ersten, eine aus der zweiten und 3 aus der dritten Untergruppe) wurden keine Blastome festgestellt. Die überlebenden Mäuse wurden am 36. Tag des Versuchs getötet. Während einer Sektion wurden bei 8 Mäusen (2 aus der ersten, 4 aus der zweiten und 2 aus der dritten Untergruppe) vereinzelte pulmonale Metastasen festgestellt. Die inneren Organe blieben ohne sichtbare Änderungen.

In der ersten und zweiten Untergruppe der Kontrollgruppe überlebten bis zum 36. Tag des Versuchs 2 Mäuse aus der ersten und 4 Mäuse aus der zweiten Untergruppe. Blastome hatten eine durchschnittliche Größe von 2,0x1,0 cm. Während der Sektion wurde eine Lungen- und Leberverletzung festgestellt.

Alle Mäuse aus der dritten Untergruppe der Kontrollgruppe starben am 12., 15., 20., 25. und 26. Tag. Bei der Untersuchung wurden blutende Rückenblastome mit einer durchschnittlichen Größe von 2,5x2,0 cm festgestellt. Eine histologische Untersuchung zeigte eine totale Lungenlysis.

Schlussfolgerungen: Das Arzneimittel besitzt eine ausgeprägte antiblastomatöse Wirkung und verursacht keine Nebenwirkungen.

### Beispiel 9

Ein kranker K. von 69 Jahren hatte als Beschwerden ein schmerzhaftes Harnlassen sowie eine Harnsperre und -rötung. Eine Ultraschalluntersuchung vom 08.08.2003 zeigte die Prostata mit einer Größe von etwa 90 cm³ und eine Geschwulst mit unscharfen Konturen und einer Größe von 35x48 mm². Eine PSA-Untersuchung vom 08.08.2003 ergab 27,9 mg/ml. Eine Punktionsbiopsie zeigte ein Adenokarzinom der Vorsteherdrüse.

Die Diagnose ergab ein Adenokarzinom der Vorsteherdrüse. Der Kranke sagte eine Operation und Chemotherapie ab. Der Kranke wurde mit dem Arzneimittel behandelt. Am 09.08.2003 wurde die Behandlung mit der 4-Chlor-2Methylphenoxyessigsäure in einer Einmaldosis von 300 g zweimal täglich während des Essens begonnen. Die Behandlung dauerte 10 Tage. In dieser Zeitperiode wurden die Schmerzen beim Harnlassen geringer, und die Urinfarbe war in Ordnung. Eine Ultraschalluntersuchung zeigte, dass die Prostata um 30 % kleiner geworden war. Die Geschwulst hatte eine Größe von 12x24 mm² und scharfe Konturen.

Danach wurde die Therapie im Laufe von 2 Wochen mit einem Gemisch aus der 4-Chlor-2-Methylphenoxyessigsäure, Kalium- und Natriumsalz in einem Verhältnis von 50:10:40 je 400 g zweimal am Tag sowie mit einem täglichen Klistieren vor dem Schlafengehen mit 2,0 g Natriumsalz der MCPA-Säure, gelöst in 50 ml Wasser, fortgesetzt. Während der nachfolgenden Untersuchung waren die krankhaften Symptome nach Aussage des Patienten vollständig verschwunden (kein schmerzhaftes Harnlassen, keine Harnsperre u.a.).

Eine Kontrolluntersuchung mit Ultraschall zeigte eine Größe der Prostata von etwa ≈ 42 cm³ und eine Geschwulst von 6x6 mm². Eine PSA-Untersuchung am 8.10.2003 ergab 4,1 mg/ml.

Dem Patienten wurde empfohlen, das Präparat 4-Chlor-2-Methylphenoxyessigsäure in einer Dosis von 200 mg zweimal am Tag während des Essens im Laufe eines Monats einzunehmen sowie tägliche Klistiere vor dem Schlafengehen mit je 2,0 g Natrium- und Kaliumsalz der Säure (im Verhältnis 50:50 Gew.-%), gelöst in 50 ml Wasser, anzuwenden.

Die Blutwerte der Laboruntersuchung sind in der folgenden Tabelle aufgeführt:

| Allgemeine Blutuntersuchung | | | |
|---|---|---|---|
| | Vor Behandlung | nach 2 Wochen | nach 2 Monaten |
| Hämoglobin, g/l | 100 | 134 | 145 |
| Erythrozyte, 1×10¹²/l | 3,5 | 4,3 | 4,7 |
| Hämoglobinindex | 0,75 | 0,85 | 0,96 |
| Leukozyte, 1 × 10⁹/l | 3,0 | 4,7 | 6,0 |
| Trombozyte, absolut (Tsd.) | 156 | 323 | 354 |
| Eosinophile, % | 3,0 | 3,0 | 2,0 |

| Neutrophile: | | | |
|---|---|---|---|
| Normalstabskernig, % | 6,0 | 6,0 | 6,0 |
| Segmentkernig, % | 69 | 71 | 72 |
| Lymphozyte, % | 21 | 22 | 26 |
| Monozyte, % | 4,5 | 6,0 | 5,8 |
| BKS mm/h | 30 | 14 | 12 |

| Biochemische Blutuntersuchung | | | |
|---|---|---|---|
| | Vor Behandlung | nach 2 Wochen | nach 2 Monaten |
| Glukose mmol/l | 3,6 | 4,9 | 4,0 |
| Harnstoff mg/dl | 43,1 | 41,1 | 38,7 |
| Harnsäure mg/dl | 5,2 | 6,3 | 6,4 |
| Albumin g/l | 39,4 | 49,6 | 50,1 |
| Protein | 81,1 | 82,9 | 88,9 |
| Kreatinin mg/dl | 0,43 | 0,39 | 0,35 |
| Aspartaminotransferase Einheiten/l | 34,0 | 28,0 | 31,0 |
| Alaninaminotransferase Einheiten/l | 77,0 | 71,4 | 55,9 |
| Gammaglutamiltransferase | 129,0 | 82,0 | 68,6 |

| Einheiten/l | | | |
|---|---|---|---|
| Laktat dehydrogenase Einheiten/l | 478,0 | 135,0 | 96,8 |

| Untersuchungsergebnisse Zell- und Humoralimmunität | | | |
|---|---|---|---|
| | Vor Behandlung | nach 2 Wochen | nach 2 Monaten |
| Immunglobulin A, g/l | 2,4 | 2,31 | 2,59 |
| Immunglobulin M, g/l | 1,9 | 1,52 | 1,65 |
| Immunglobulin G, g/l | 11,0 | 10,5 | 10,6 |
| T-Lymphozyte, % | 54,0 | 62,0 | 68,0 |
| B-Lymphozyte, % | 14,0 | 21,0 | 32,0 |
| Latex-Phagozytose,% | 54 | 55 | 58 |
| TNF | 15,6 | 23,0 | 32 |
| TH (Helper-Zelle), % | 24,0 | 31,0 | 25,0 |

### Beispiel 10

Eine kranke A. von 43 Jahren litt an einer Exstirpation der Gebärmutter und des - anhanges wegen eines Karzinoms des rechten Eierstocks im Jahr 2000. Nach der Operation erfolgten 2 Jahre lang Chemotherapiekuren. Ende Juli 2002 traten Rektum- und Tenesmusschmerzen auf. Ein Computertomogramm vom 18.08.2002 zeigte eine Geschwulst mit einer Größe von 17,0x15,9 cm² zwischen dem Mastdarm und der Harnblase, die deren Wände durchwuchs. Es wurde ein Geschwulstrezidiv diagnostiziert. Angesichts der uneffektiven Chemotherapie wurde eine Behandlung mit dem Präparat durchgeführt: im Laufe von 14 Tagen wurden 400 mg des Kaliumsalzes der 4-Chlor-2-Methylphenoxyessigsäure dreimal pro Tag eingenommen, und es wurden tägliche Klistiere mit 2,0 g Gemisch aus der 4-Chlor-2-Methylphenoxyessigsäure und dem Kaliumsalz dieser Säure (im Verhältnis 90:10 Gew.-%) angewendet, danach im Laufe von 21 Tagen ein Gemisch aus je 500 mg Kalium- und Natriumsalz der Säure (im Verhältnis 25:75 Gew.-%) zweimal am Tag und tägliche Klistiere mit 2,0 g Gemisch aus der 4-Chlor-2-Methylphenoxyessigsäure, dem Kalium- und Natriumsalz dieser Säure (im Verhältnis 25:25:50 Gew.-%).

Am 3. Tag der Behandlung wurden die Schmerzen schwächer, und der Harn- und Defäkationsdrang wurde seltener. Eine Ultraschalluntersuchung nach 2 Wochen ergab eine Geschwulstgröße von 7,4×5,7 cm², nach weiteren 2 Wochen 1,2×2,3 cm² sowie eine scharfe Grenze zwischen der Geschwulst und den Mastdarm- und Harnblasenwänden. Eine Kontrolluntersuchung mit Ultraschall nach 2 Monaten zeigte ein rundes, scharf begrenztes Gebilde mit einer Größe von 0,6x0,5 cm², in das eine dicke, pulsierende Arterie hineinging. Eine zusätzliche Untersuchung zeigte einen genügenden Zustand, während eine Ultraschalluntersuchung vom 23.02.2004 ein scharf begrenztes Gebilde mit einer Größe von 0,2×0,1 cm² ohne sichtbaren Blutkreislauf zeigte. Es fand sich kein Exsudat im kleinen Becken.

Die Blutwerte der Laboruntersuchung sind in der folgenden Tabelle aufgeführt:

| Allgemeine Blutuntersuchung | | | |
|---|---|---|---|
| | Vor Behandlung | nach 2 Wochen | nach 2 Monaten |
| Hämoglobin, g/l | 97 | 123 | 149 |
| Erythrozyte, 1 × 10¹²/l | 2,5 | 3,3 | 4,7 |
| Hämoglobinindex | 0,75 | 1,0 | 1,0 |
| Leukozyte, 1 × 10⁹/l | 3,0 | 4,7 | 5,5 |
| Trombozyte, absolut (Tsd.) | 164 | 223 | 259 |
| Eosinophile, % | 1,0 | 2,0 | 1,0 |

| Neutrophile: | | | |
|---|---|---|---|
| Normalstabskernig, % | 5,7 | 5,9 | 6,0 |
| Segmentkernig, % | 71 | 69 | 68 |
| Lymphozyte, % | 22 | 24 | 25 |
| Monozyte, % | 5,5 | 5,0 | 5,9 |
| BKS mm/h | 33 | 16 | 10 |

| Biochemische Blutuntersuchung | | | |
|---|---|---|---|
| | Vor Behandlung | nach 2 Wochen | nach 2 Monaten |
| Glukose mmol/l | 4,6 | 5,9 | 5,0 |
| Harnstoff mg/dl | 42,5 | 44,0 | 28,9 |
| Harnsäure mg/dl | 4,9 | 6,8 | 5,4 |
| Albumin g/l | 40,4 | 47,8 | 50,4 |
| Protein g/l | 61,9 | 71,6 | 81,8 |
| Kreatinin mg/dl | 0,42 | 0,35 | 0,34 |
| Aspartataminotransferase Einheiten/l | 33,0 | 31,0 | 28,0 |
| Alaninaminotransferase Einheiten/l | 77,0 | 71,4 | 55,9 |
| Gammaglutamiltransferase Einheiten/l | 129,0 | 82,0 | 68,6 |
| Laktat dehydrogenase Einheiten/l | 543,5 | 335,0 | 126,8 |

| Untersuchungsergebnisse Zell- und Humoralimmunität | | | |
|---|---|---|---|
| | Vor Behandlung | nach 2 Wochen | nach 2 Monaten |
| Immunglobulin A, g/l | 2,1 | 2,30 | 2,29 |
| Immunglobulin M, g/l | 1,6 | 1,5 | 1,75 |
| Immunglobulin G, g/l | 10,9 | 11,0 | 10,8 |
| T-Lymphozyte, % | 56,0 | 61,0 | 65,0 |
| B-Lymphozyte, % | 12,0 | 19 | 31,0 |
| Latex-Phagozytose,% | 51 | 55 | 56 |
| TNF | 14,9 | 21,0 | 34,5 |
| TH (Helper-Zelle), % | 23,0 | 29,0 | 26,0 |

### Beispiel 11

Einer kranken U. von 35 Jahren wurde NHL (Non-Hogkin-Lymphom) diagnostiziert. Seit 1994 erfolgten eine Einnahme von Chemopräparaten und Bestrahlungsbehandlungen. Der Zustand verschlechterte sich trotz einer massiven entzündungshemmenden und Hormon-Therapie. Die Lymphknoten waren stark vergrößert (die größten hatten einen Durchmesser von 18-20 cm); Atemnot und Schwäche traten auf.

Eine Behandlung mit der 4-Chlor-2-Methylphenoxyessigsäure und deren Derivaten erfolgte. Ab dem 12.02.2003 nahm die Kranke das Präparat in einer Menge von je 400 mg zweimal am Tag per os während des Essens ein, das ein Gemisch aus der 4-Chlor-2-Methylphenoxyessigsäure und dem Natriumsalz dieser Säure (in einem Verhältnis von 30:70 Gew.-%) enthielt. In zwei Wochen verbesserte sich der Zustand. Die Atemnot verschwand. Der Appetit kam zurück. Die sichtbaren Lymphknoten wurden um den Faktor 1,5-2 kleiner.

Nach drei Wochen wurde das Präparat in einer Menge von je 300 mg zweimal am Tag zur Einnahme während des Essens verschrieben, das ein Gemisch aus Natrium- und Lithiumsalz der 4-Chlor-2-Methylphenoxyessigsäure (im Verhältnis 75:25 Gew.-%) enthielt.

Nach 1,5 Monaten seit dem Behandlungsbeginn waren die Lymphknoten vollständig (durch Ultraschalluntersuchung bestätigt) verschwunden. Der Zustand war genügend, und es traten keine Beschwerden auf. Eine Kontrolluntersuchung nach einem Jahr ergab keine Beschwerden und keine Rezidive.

Die Blutwerte der Laboruntersuchung sind in der folgenden Tabelle aufgeführt:

| Allgemeine Blutuntersuchung | | | |
|---|---|---|---|
| | Vor Behandlung | nach 2 Wochen | nach 2 Monaten |
| Hämoglobin, g/l | 130 | 134 | 145 |
| Erythrozyte, 1 × 10¹²/l | 3,5 | 4,3 | 4,7 |
| Hämoglobinindex | 1,05 | 1,05 | 1,3 |
| Leukozyte, 1 × 10⁹/l | 3,0 | 4,7 | 5,5 |
| Trombozyte, absolut (Tsd.) | 140 | 325 | 410 |
| Eosinophile, % | 3,0 | 3,0 | 1,0 |

| Neutrophile: | | | |
|---|---|---|---|
| Normalstabskernig, % | 5,1 | 5,0 | 4,5 |
| Segmentkernig, % | 62 | 56 | 54 |
| Lymphozyte, % | 24 | 21 | 25 |
| Monozyte, % | 0,5 | 0,4 | 5,0 |
| BKS mm/h | 43 | 32 | 9 |

| Biochemische Blutuntersuchung | | | |
|---|---|---|---|
| | Vor Behandlung | nach 2 Wochen | nach 2 Monaten |
| Glukose mmol/l | 3,1 | 4,7 | 4,9 |
| Harnstoff mg/dl | 44,1 | 44,1 | 38,0 |
| Harnsäure mg/dl | 6,2 | 8,3 | 7,4 |
| Albumin g/l | 37,4 | 49,6 | 50,1 |
| Protein g/l | 80,1 | 82,5 | 82,9 |
| Kreatinin mg/dl | 0,45 | 0,31 | 0,35 |
| Aspartataminotransferase Einheiten/l | 36,0 | 29,0 | 30,0 |
| Alaninaminotransferase Einheiten/l | 75,0 | 65,7 | 46,9 |
| Gammaglutamiltransferase Einheiten/l | 107,0 | 98,0 | 45,6 |
| Laktat dehydrogenase Einheiten/l | 459,0 | 230,0 | 217,4 |

| Untersuchungsergebnisse der Zell- und Humoralimmunität | | | |
|---|---|---|---|
| | Vor Behandlung | nach 2 Wochen | nach 2 Monaten |
| Immunglobulin A, g/l | 2,05 | 2,32 | 2,53 |
| Immunglobulin M, g/l | 1,9 | 1,57 | 1,56 |
| Immunglobulin G, g/l | 11,0 | 11,5 | 11,6 |
| T-Lymphozyte, % | 54,0 | 65,0 | 69,0 |
| B-Lymphozyte, % | 15,0 | 24,0 | 42,0 |
| Latex-Phagozytose,% | 43 | 80 | 67 |
| TNF | 15,6 | 23,0 | 32,3 |
| TH (Helper-Zelle), % | 26,0 | 30,0 | 28,0 |
| TS (Suppressor-Zelle), % | 23,0 | 28,0 | 31,0 |

### Beispiel 12

Einem kranken N. von 27 Jahren wurde Hepatitis C, Hepatargie, Aszites diagnostiziert. Der Kranke wurde 1989 durch medizinische Werkzeuge oder eine Hämatransfusion nach einer Bauchoperation wegen Bauchverletzung mit dem Virus von Hepatitis C infiziert. Die ersten krankhaften Symptome erschienen nach 9 Jahren seit der Infizierung, nämlich Schmerzen in den rechten Unterrippen, Gelbsucht, schlechte biochemische Blutwerte. Nach der Behandlung im Krankenhaus verbesserte sich der Zustand. Rezidiv chronische Hepatitis trat nach der Grippe im Jahr 2001 auf. Ferner traten Aszites und Beinödem, subfebrile Temperatur und Gelbsucht auf. Im November 2002 wurde dem Kranken eine Lebertransplantation angeboten, die er absagte.

Im Januar 2003 wurde die Behandlung mit dem Arzneimittel begonnen. Im Laufe von 2 Wochen nahm der Patient die 4-Chlor-2-Methylphenoxyessigsäure in einer Menge von je 250 mg zweimal am Tag ein und danach das Gemisch aus der Säure und den Kalium-, Natrium- und Lithiumsalzen dieser Säure in gleichen Verhältnissen in einer Menge von je 400 mg dreimal am Tag während des Essens. Im Laufe eines Monats verringerte sich die Bluttransaminase wesentlich. Die Gelbsüchtigkeit wurde geringer. Hautjucken, Schmerzen in den rechten Unterrippen und eine vermehrte Schweißabsonderung waren verschwunden. Nach einem Monat wurde die Einmaldosis des Präparates verdoppelt. Nach zwei Monaten war der Zustand genügend. Es traten kein Beinödem und keine Aszites auf. Die Haut war gelblich, und die Augapfelhäute waren hell.

Dem Kranken wurde empfohlen, das Präparat in Form eines Gemisches aus Kalium- und Lithiumsalz der 4-Chlor-2-Methylphenoxyessigsäure im gleichen Verhältnis zu je 250 mg ein- bis zweimal am Tag einen Monat lang einzunehmen.

Die Blutwerte der Laboruntersuchung sind in der folgenden Tabelle aufgeführt:

| Allgemeine Blutuntersuchung | | | |
|---|---|---|---|
| | Vor Behandlung | nach 2 Wochen | nach 2 Monaten |
| Hämoglobin, g/l | 100 | 123 | 149 |
| Erythrozyte, 1 x 10¹²/l | 4,5 | 4,3 | 5,0 |
| Hämoglobinindex | 0,85 | 1,0 | 1,0 |
| Leukozyte, 1 x 10⁹/l | 8,0 | 4,7 | 6,1 |
| Trombozyte, absolut (Tsd.) | 104 | 127 | 224 |
| Eosinophile, % | 3,0 | 4,0 | 1,0 |

| Neutrophile: | | | |
|---|---|---|---|
| Normalstabskernig, % | 4,6 | 4,9 | 5,0 |
| Segmentkernig, % | 67 | 68 | 65 |
| Lymphozyte, % | 20 | 23 | 23 |
| Monozyte, % | 4,5 | 4,0 | 4,9 |
| BKS mm/h | 43 | 24 | 12 |

| Biochemische Blutuntersuchung | | | |
|---|---|---|---|
| | Vor Behandlung | nach 2 Wochen | nach 2 Monaten |
| Natrium mmol/l | 139 | 143 | 143 |
| Kalium mmol/l | 3,0 | 4,2 | 4,5 |
| Glukose mmol/l | 4,6 | 5,9 | 5,0 |
| Harnstoff mg/dl | 42,5 | 44,0 | 28,9 |
| Harnsäure mg/dl | 4,9 | 6,8 | 5,4 |
| Bilirubin, mg/dl | 2,9 | 1,7 | 0,8 |
| Albumin g/dl | 2,0 | 4,7 | 5,4 |
| Protein g/dl | 6,1 | 7,1 | 8,4 |
| Kreatinin mg/dl | 0,42 | 0,35 | 0,34 |
| Aspartataminotransferase Einheiten/l | 73,0 | 51,0 | 28,0 |
| Alaninaminotransferase Einheiten/l | 77,0 | 71,4 | 43,9 |
| Gammaglutamiltransferase Einheiten/l | 129,0 | 82,0 | 68,6 |
| Laktat dehydrogenase Einheiten/I | 543,5 | 335,0 | 247,0 |

## Patentansprüche

1. Arzneimittel mit immunmodulierenden, entzündungshemmenden und antiblastomatösen Eigenschaften sowie einer Antivirusaktivität,
**dadurch gekennzeichnet,**
**dass** es die 4-Chlor-2-Methylphenoxyessigsäure mit der Strukturformel: 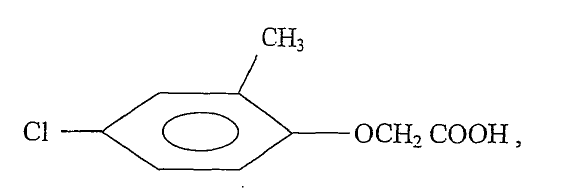 und/oder pharmakologisch vertretbare Derivate dieser Säure, beispielsweise Salze dieser Säure von Alkalimetallen oder Gemische dieser Salze bzw. Gemische aus der 4-Chlor-2-Methylphenoxyessigsäure und diesen Derivaten aufweist.

2. Arzneimittel nach Anspruch 1,
**dadurch gekennzeichnet,**
**dass** das Derivat der 4-Chlor-2-Methylphenoxyessigsäure das Kaliumsalz dieser Säure darstellt.

3. Arzneimittel nach Anspruch 1,
**dadurch gekennzeichnet,**
**dass** das Derivat der 4-Chlor-2-Methylphenoxyessigsäure das Natriumsalz dieser Säure darstellt.

4. Arzneimittel nach Anspruch 1,
**dadurch gekennzeichnet,**
**dass** das Derivat der 4-Chlor-2-Methylphenoxyessigsäure das Lithiumsalz dieser Säure darstellt.

5. Arzneimittel nach Anspruch 1,
**dadurch gekennzeichnet,**
**dass** das Derivat der 4-Chlor-2-Methylphenoxyessigsäure ein Gemisch aus 1,0-98,0 Gew.-% Kaliumsalz und restlichem Natriumsalz darstellt.

6. Arzneimittel nach Anspruch 1,
**dadurch gekennzeichnet,**
**dass** das Derivat der 4-Chlor-2-Methylphenoxyessigsäure ein Gemisch aus 1,0-98,0 Gew.-% Kaliumsalz und restlichem Lithiumsalz darstellt.

7. Arzneimittel nach Anspruch 1,
**dadurch gekennzeichnet,**
**dass** das Derivat der 4-Chlor-2-Methylphenoxyessigsäure ein Gemisch aus 1,0-98,0 Gew.-% Natriumsalz und restlichem Lithiumsalz darstellt.

8. Arzneimittel nach Anspruch 1,
**dadurch gekennzeichnet,**
**dass** das Derivat der 4-Chlor-2-Methylphenoxyessigsäure ein Gemisch aus 1,0-55,0 Gew.-% Kalium-, 1,0-85,0 Natrium- und restlichem Lithiumsalz darstellt.

9. Arzneimittel nach Anspruch 1,
**dadurch gekennzeichnet,**
**dass** das Gemisch aus der 4-Chlor-2-Methylphenoxyessigsäure und deren Derivaten in einem Verhältnis von 1,0-98,0 Gew.-% 4-Chlor-2-Methylphenoxyessigsäure und restlichen Derivaten ausgeführt.
